# EUROPEAN PATENT SPECIFICATION

(11) **EP 2 310 528 B1**
(45) Date of publication and mention of the grant of the patent: **20.11.2013**
(21) Application number: 09802498.7
(22) Date of filing: 27.07.2009
(51) Int. Cl.: C12Q 1/68

(54) **A GENETIC MARKER TEST FOR BRACHYSPINA AND FERTILITY IN CATTLE**
GENETISCHER MARKER ZUM TESTEN VON RINDERN AUF BRACHYSPINA UND FRUCHTBARKEIT
MARQUEUR GÉNÉTIQUE POUR TESTER LE BRACHYSPINA ET LA FERTILITÉ CHEZ LES BOVINS

(30) Priority: 29.07.2008 EP 08013608; 30.06.2009 WO PCT/EP2009/058190
(43) Date of publication of application: 20.04.2011
(73) Proprietor: Université de Liège, 4031 Angleur (BE); University of Copenhagen, 1017 Copenhagen K (DK)
(72) Inventor: GEORGES, Michel, 4161 Villers-aux-Tours (BE); COPPIETERS, Wouter, 3401 Landen (BE); CHARLIER, Carole, 4140 Sprimont (BE); AGERHOLM, Jørgen Steen, 2650 Hvidovre (DK); FREDHOLM, Merete, 2800 Lyngby (DK)
(74) Representative: Vossius & Partner
(86) International application number: PCT/EP2009/059675
(87) International publication number: WO 2010/012690

(56) References cited:
- WO-A-2007/112490
- ANONYMOUS: "brachyspina marker test" DAIRY FARMER, 19 September 2008 (2008-09-19), page 30, XP001538659
- WHITLOCK B K ET AL: "Heritable bovine fetal abnormalities" THERIOGENOLOGY, vol. 70, no. 3, 1 August 2008 (2008-08-01), pages 535-549, XP022776686 LOS ALTOS, CA, US ISSN: 0093-691X [retrieved on 2008-06-02] -& WHITLOCK B K ET AL: "Heritable bovine fetal abnormalities" THERIOGENOLOGY, [Online] vol. 70, no. 3, 2 June 2008 (2008-06-02), XP002508789 LOS ALTOS, CA, US Retrieved from the Internet: URL:http://www.sciencedirect.com/> [retrieved on 2008-10-08]
- CHARLIER CAROLE ET AL: "Highly effective SNP-based association mapping and management of recessive defects in livestock" NATURE GENETICS, vol. 40, no. 4, April 2008 (2008-04), pages 449-454, XP002507256 ISSN: 1061-4036 cited in the application
- AGERHOLM JORGEN S ET AL: "Brachyspina syndrome in a Holstein calf." JOURNAL OF VETERINARY DIAGNOSTIC INVESTIGATION : OFFICIAL PUBLICATION OF THE AMERICAN ASSOCIATION OF VETERINARY LABORATORY DIAGNOSTICIANS, INC JUL 2006, vol. 18, no. 4, July 2006 (2006-07), pages 418-422, XP002507255 ISSN: 1040-6387 cited in the application
- AGERHOLM JORGEN S ET AL: "Familial occurrence of Danish and Dutch cases of the bovine brachyspina syndrome" BMC VETERINARY RESEARCH, BIOMED CENTRAL, LONDON, GB, vol. 3, no. 1, 8 May 2007 (2007-05-08), page 8, XP021025960 ISSN: 1746-6148 cited in the application
- TESTONI S ET AL: "Brachyspina syndrome in two Holstein calves" VETERINARY JOURNAL, BAILLIERE TINDALL, LONDON, GB, vol. 177, no. 1, 1 July 2008 (2008-07-01), pages 144-146, XP022690043 ISSN: 1090-0233 [retrieved on 2008-06-02]

## Description

### Field of the invention

This invention relates to methods for the detection of bovine that are affected by or carriers of Brachyspina (BS), an inherited defect with autosomal recessive inheritance. The present invention provides a method for determining whether a bovine is affected by or carrier of BS by analyzing its genomic DNA or its RNA. The method includes obtaining a sample of material containing genomic DNA or RNA from the bovine, genotyping said nucleic acid for one or more SNP markers defining the BS diagnostic haplotype, and ascertaining whether the said animal carries the diagnostic haplotype indicating that it is affected by or carrier of BS.

### Description of the background art

Intense selection for desired production traits in livestock often results in an increased rate of inbreeding. This causes the frequent emergence of inherited defects which sometimes affect such a high proportion of the population that it becomes a major issue. Examples of genetic defects that have caused major problems in livestock are bovine leucocyte adhesion deficiency (BLAD) (I) and congenital vertebral malformation (CVM) (2).

Most of these disorders are characterized by a recessive mode of inheritance: animals can be homozygous for the normal allele (+/+), homozygous for the defective allele (D/D), or heterozygous (+/D). D/D animals are afflicted by the disease, while +/+ and +/D animals are generally healthy. +/D animals, however, are said to be "carriers" and will transmit the D allele to halve their offspring. The mating between two carrier animals will produce one fourth of affected D/D offspring.

If one could identify +/D animals despite the fact that they do not exhibit the symptoms of the disease, it would be possible to eliminate them from breeding programs or at least avoid matings between carriers. This would allow immediate eradication of the genetic defect from the population and avoid the economic losses associated with it.

It has recently become possible to rapidly identify the genes underlying genetic defects and the mutations in them which cause an allele to be D(efective) (3). The most commonly used procedure is positional cloning which comprises two steps. In a first instance the D(efective) gene is localized in the genome via the identification of a chromosome segment that is shared "homozygous by descent" by all affected individuals in a given population. To that end affected individuals are genotyped for tens of thousands of polymorphic Single Nucleotide Polymorphisms (SNPs) scattered throughout the genome. The mutation causing the D allele appeared on a specific chromosome which is characterized by a unique "haplotype", i.e. combination of alleles at the SNPs surrounding the mutation. Affected individuals have inherited the causal mutation from both parents and with it the diagnostic haplotype, hence the shared homozygosity in the corresponding chromosome region. In a second instance, the causal gene and mutation are identified by detailed molecular examination of the chromosome region shared "homozygous by descent".

From a diagnostic point of view, the detection of carrier +/D individuals can either be done on the basis of the detection of the diagnostic haplotype, or better the causal mutation.

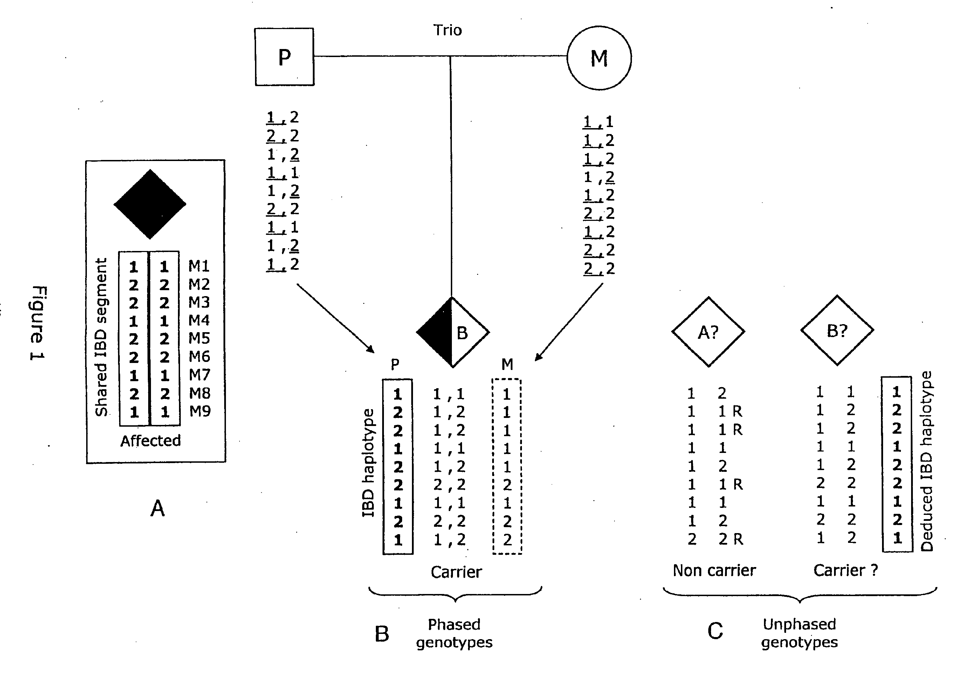

Mammals are diploid, meaning that they carry two haplotypes at each chromosomal region, one paternal and one maternal. The superposition of the two haplotypes may blurry the interpretation as illustrated in Figure 1. To determine if an individual carries a defined haplotype at a given map position one needs to untangle the paternal and maternal haplotypes, a procedure known as "phasing". This can either be done on the basis of available parental genotypes, genotypes of offspring, population-wide linkage disequilibrium or a combination of these different sources of information. Note that even if accurate phasing is impossible, the presence of a specific haplotype can often be excluded just from the analysis of the unphased genotypes.

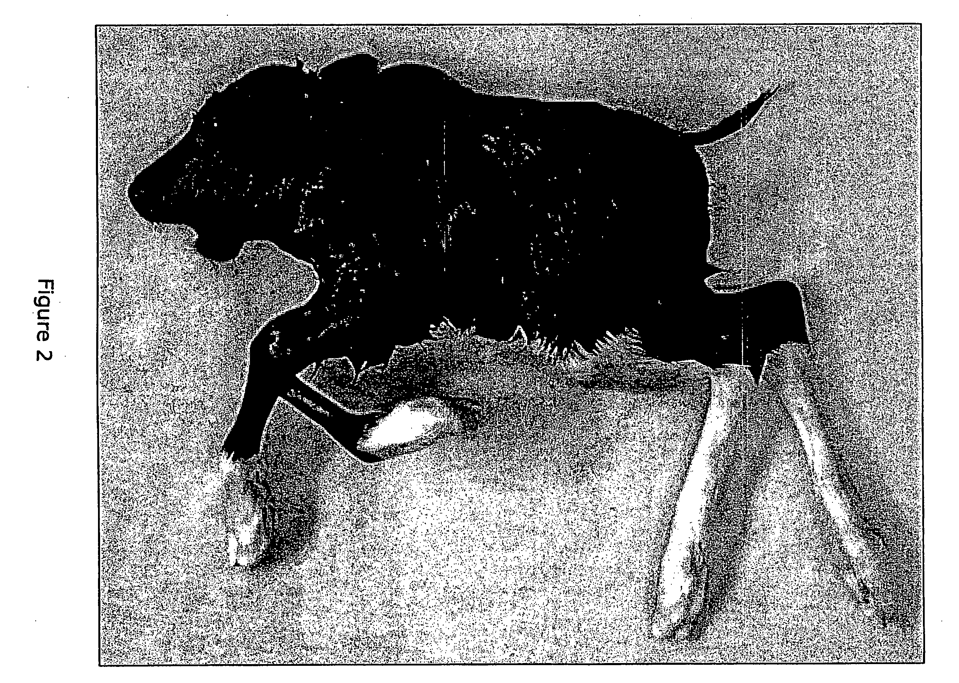

In 2006, a beforehand unknown congenital lethal syndrome was reported in a Danish Holstein calf (Agerholm JS, McEvoy F, Arnbjerg J: Brachyspina syndrome in a Holstein calf. J Vet Diagn Invest 2006, 18:418-422). The syndrome was characterized by severely reduced body weight, growth retardation, severe vertebral malformations associated with a significant shortening of the spine (brachyspina) and long and slender limbs. In addition, affected animals exhibit inferior brachygnatism as well as malformation of the inner organs, in particular the heart, kidneys and testis (4) as shown in Figure 2. The cause of the syndrome could not be determined. Since the calf was the result of artificial breeding between genetically related and phenotypically normal animals and as it was the only affected calf occurring in a herd of 96 adult females, one could speculate that the incident occurred by transmission by descent of a defective recessive allele from a common ancestor. Further cases could then be identified through the Danish Bovine Genetic Disease Programme (Agerholm JS, Basse A, Christensen K: Investigations on the occurrence of hereditary diseases in the Danish cattle population 1989-1991. Acta Vet Scand 1993, 34:245-253). But again, the cause of the disease could not be determined. A review of Whitlock et al. (Theriogenology, 2008, 70 (3): 535-549) discusses the etiologies for congenital bovine fetal anomalies and reports of six cases ofbrachyspina syndrome in the literature prior brachyspina.

### Summary of the invention

In view of the above, the technical problem underlying the present invention was to provide means and methods that allow for a selective and convenient diagnosis of brachyspina or of carrier-status for this disease in cattle.

The solution to said technical problem is achieved by the embodiments characterized in the claims.

The present invention provides for the first time the chromosomal localisation of the gene causing brachyspina, and SNP haplotypes are described which are "associated" with the D allele and which can be utilized for highly accurate detection of +/D carriers.

Thus, the present invention provides in a first embodiment:
a method for determining whether a bovine is affected by or carrier of brachyspina (BS) by analyzing its genomic DNA, the method comprising the steps of:
   a) extracting the DNA from a sample of biological material containing said genomic DNA obtained from the bovine,
   b)i) genotyping said DNA for SNPs located in the interval between nucleotide positions 20156961 and 22499122 on bovine chromosome 21, Btau_4.0, and
   c) ascertaining whether said animal carries the brachyspina-risk haplotype.

The term "bovine" in accordance with the present invention encompasses all cattle or cattle breeds from the species bos taurus. In a preferred embodiment of the methods of the present invention the bovine is selected from the group consisting of Holstein, Friesian and Holstein-Friesian Cross breeds, British and /or Dutch Friesian.

The term "carrier of brachyspina (BS)" refers to a bovine that carries a mutation causing the Brachyspina defect on one of its chromosomes (whether inherited from sire or dam), and a wild-type allele on the other chromosome. The mutation causing brachyspina is located on bovine chromosome 21 in the interval between nucleotide positions 20156961 and 22499122 (Btau_4.0).

The term "sample" or "biological sample" according to the present invention refers to any material containing nucleated cells from said bovine to be tested. In a preferred embodiment the biological sample to be used in the methods of the present invention is selected from the group consisting of blood, sperm, hair roots, milk, body fluids as well as tissues including nucleated cells.

DNA extraction / isolation and purification methods are well-known in the art and can be applied in the present invention. Standard protocols for the isolation of genomic DNA are inter alia referred to in Sambrook, J., Russell, D.W., Molecular Cloning: A Laboratory Manual, the third edition, Cold Spring Harbor Laboratory Press, Cold Spring Harbor, New York, 1.31-1.38. 2001 and Sharma. R.C., et al.. A rapid procedure for isolation of RNA-free genomic DNA from mammalian cells, BioTechniques, 14, 176-178, 1993.

According to the present invention the term "SNP" refers to a single nucleotide polymorphism at a particular position in the bovine genome that varies among a population of individuals. SNPs can be identified by their location within the disclosed particular sequence, i.e. within the interval of base positions 20156961 and 22499122 on bovine chromosome 21 (Btau_4.0) or their name as shown in Table 1. The SNPs identified as being indicative for the diagnosis of brachyspina according to the present invention are shown in Table 1. For example, the SNP BTA-51815 of Table 1 indicates that the nucleotide base (or the allele) at nucleotide position 20210931 on chromosome 21 of the reference sequence as referred to herein may be either adenine (A) or guanine (G). The allele associated with or indicative for brachyspina is in case of SNP BTA-51815 of Table 1 guanine (G).

The term "genotyping said DNA for SNPs" in accordance with the present invention refers to a method for determining or identifying whether a particular nucleotide sequence is present in a DNA sample. There are several methods known by those skilled in the art, e.g. (5) for determining whether such nucleotide sequence is present in a DNA sample. These include the amplification of a DNA segment encompassing the SNPs composing the haplotype by means of the polymerase chain reaction (PCR) or any other amplification method, and interrogate the SNP position by means of allele specific hybridization, or the 3'exonuclease assay (Taqman assay), or fluorescent dye and quenching agent-based PCR assay, or the use of allele-specific restriction enzymes (RFLP-based techniques), or direct sequencing, or the oligonucleotide ligation assay (OLA), or pyrosequencing, or the invader assay, or minisequencing, or DHPLC-based techniques, or single strand conformational polymorphism (SSCP), or allele-specific PCR, or denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, extension based assays, ARMS (Amplification Refractory Mutation System), ALEX (Amplification Refractory Mutation Linear Extension), SBCE (Single base chain extension), a molecular beacon assay, invader (Third wave technologies), a ligase chain reaction assay, 5'-nuclease assay-based techniques, hybridization capillary array electrophoresis (CAE), protein truncation assay (PTT), immunoassays and solid phase hybridization (dot blot, reverse dot blot, chips). This list of methods is not meant to be exclusive, but just to illustrate the diversity of available methods. Some of these methods can be performed in microarray format (microchips) or on beads.

Further disclosed is the use of primers or primer pairs, wherein the primers or primer pairs hybridize(s) under stringent conditions to the DNA of claim 1 comprising the interval between nucleotide position 20156961 and 22499122 on bovine chromosome 21 or to the complementary strand thereof.

Preferably, the primers have a length of at least 14 nucleotides such as 17 or 21 nucleotides.

Herein disclosed are primers that actually bind to the position of the SNPs as referred to in Table 1. Such an allele specific oligonucleotide is typically an oligonucleotide of at least 14 to 21 nucleotide bases in length designed to detect a difference of a single base in the target's genetic sequence of the bovine to be tested. One or more specific primers can be applied in order to identify more than a single SNP as referred to herein. As a consequence, when binding is performed under stringent conditions, such primer or such primers is/are useful to distinguish between different polymorphic variants as binding only occurs if the sequences of the primer and the target have full complementarity. It is further preferred that the primers have a maximum length of 24 nucleotides. Such primers can be coupled with an appropriate detection method such as an elongation reaction or an amplification reaction which may be used to differentiate between the polymorphic variants and then draw conclusions with regard to, e.g., the predisposition of the cattle or calf under investigation for brachyspina.

Hybridisation is preferably performed under stringent or highly stringent conditions. "Stringent or highly stringent conditions" of hybridization are well known to or can be established by the person skilled in the art according to conventional protocols. Appropriate stringent conditions for each sequence may be established on the basis of well-known parameters such as temperature, composition of the nucleic acid molecules, salt conditions etc.: see, for example, Sambrook et al., "Molecular Cloning, A Laboratory Manual"; CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson, 3 to 15. Typical (highly stringent) conditions comprise hybridization at 65°C in 0.5xSSC and 0.1% SDS or hybridization at 42°C in 50% formamide, 4xSSC and 0.1% SDS. Hybridization is usually followed by washing to remove unspecific signals. Washing conditions include conditions such as 65°C, 0.2xSSC and 0.1% SDS or 2xSSC and 0,1% SDS or 0,3xSSC and 0,1% SDS at 25°C - 65°C.

The term "base positions 20156961 and 22499122 on bovine chromosome 21" refers to the bos taurus reference sequence Btau_4.0 which can be retrieved from e.g. the UCSC, Ensembl, and NCBI genome browsers. The Btau_4.0 was generated by the Atlas genome assembly system at Baylor College of Medicine Human Genome Sequencing Center. The sequencing strategy combined BAC shotgun reads with whole genome shotgun reads from small insert libraries as well as BAC end sequences. The nucleotide sequence for base positions 20156961 to 22499122 of bovine chromosome 21 (Btau_4.0) as referred to herein is shown in SEQ ID N°1.

The term "brachyspina-risk haplotype" as used herein refers to allelic combinations at one or more SNPs mapping to base positions 20156961 and 22499122 on bovine chromosome 21, that are associated with BS. 104 of these SNPs, which can be genotyped with the two most commonly used Illumina^{™} SNP arrays (references 3 and 6), are listed in Table 1 which also reports for each SNP the BS-associated allele and its frequency in the Holstein-Friesian population. This SNP list is not meant to be exhaustive. Sequencing of DNA samples from affected individuals and/or carriers and controls, within the corresponding interval or even flanking regions, using methods which are well known by those skilled in the art, will reveal additional SNPs exhibiting varying degrees of association with BS and could be used to perform diagnosis for BS in the same way as performed in the examples reported in this invention on the basis of SNPs listed in Table 1. The specificity and sensitivity of the diagnostic test will increase with the number of SNPs included in the analysis. However, we have demonstrated that the maximum specificity/sensitivity is readily achieved with a relatively small number of SNPs. Thus, in a preferred embodiment of the present invention a single SNP as referred to above is indicative for the diagnosis of BS. In another embodiment of the present invention at least 5, SNPs as identified herein in Table 1 can be applied for the diagnosis of BS. The SNPs to be preferably analyzed in the diagnostic method of the present invention are those that have the lowest frequency in the general Holstein-Friesian population. Thus one can sort the SNPs in Table 1 by increasing allelic frequency and include SNP for a diagnostic test by starting from the top of the list. We have shown that even when working with "unphased" data (see hereafter) the seven best SNPs provide optimal sensitivity/specificity.

In order to determine if an individual carries a defined haplotype at a given map position of the sequence it can be necessary to untangle the paternal and maternal haplotypes, a procedure known as "phasing". Since bovines are diploid, haplotype (or phase) information is not immediately available. As an example, two SNPs lying on the same chromosome, both with alleles C and G. If both SNPs are observed as heterozygous, it is unclear whether one chromosome contains allele C at both loci and the other chromosome contains allele G in both loci, or whether one chromosome contains allele C at the first locus and allele G at the second locus and the other chromosome contains alleles G and C, respectively.

Obtaining the haplotypes is the first step for many types of analysis of SNP variations. The construction of haplotypes from the diploid genotype information (i.e., phasing the genotypes) can be done on the basis of available parental genotypes, genotypes of offspring, population-wide linkage disequilibrium or a combination of these different sources of information. Alleles of SNPs that are physically located in close proximity to each other on a chromosome are often correlated ("linkage disequilibrium") with each other. Thus, within most short regions, there is limited genetic variability, and only a small number of allele sequences (haplotypes) exist in a population. The haplotype structure of a given region depends on evolutionary and population genetic factors such as mutation and recombination rates, selection, and population history. Computer programs have been developed to estimate and assign phase from diploid genotype data (e.g. Stephens et al. 2001, Am. J. Hum. Genet. 68: 978-989; Halperin and Eskin 2004; Bioinformatics 20:1842-1849 and Druet et al., unpublished).

Therefore, in a preferred embodiment of the present invention, the above described method of the present invention comprises the additional step b)ii), resulting in a method for determining whether a bovine is affected by or a carrier of brachyspina (BS) by analyzing its genomic DNA, the method comprising the steps of :
a) extracting the DNA from a sample of biological material containing said genomic DNA obtained from the bovine,
b)i) genotyping said DNA for SNPs located in the interval between nucleotide positions 20156961 and 22499122 on bovine chromosome 21, Btau_4.0, and
b)ii) phasing the SNP genotype of said bovine.
c) ascertaining whether said animal carries the brachyspina-risk haplotype.

In a further embodiment of the present invention a method is provided for determining whether a bovine is a carrier of brachyspina (BS) by analyzing its RNA, the method comprising the steps of:
a) extracting the RNA from a sample of biological material containing said RNA obtained from the bovine,
b)i) genotyping said RNA for SNPs located in the interval between nucleotide positions 20156961 and 22499122 on bovine chromosome 21, Btau_4.0, and
b)ii) phasing the SNP genotype of said bovine.
c) ascertaining whether said animal carries the brachyspina-risk haplotype.

### "RNA" as referred to herein encompasses all types of RNA.

Techniques well known in the art in order to allow for the isolation of total RNA, mitochondrial RNA, or messenger RNA. The person skilled in the art can select a suitable extraction method without further ado depending on the nature of the sample to be tested.

If a sample containing RNA is to be used as a template for an amplification reaction, it will be necessary to transcribe said RNA in cDNA before amplification can be carried out. Again, techniques for doing so are well known to the person skilled in the art. As an example the RNA may be purified with RNeasy^{™} Mini Kit (Qiagen). The RNA will then be reversely transcribed to cDNA using, e.g. the SuperScript^{™} Choice System (Invitrogen).

Furthermore, the RNA, or genomic DNA obtained from the bovine subject may be sequenced to identify mutations which may give characteristic fingerprints of mutations in the nucleic acid sequence as referred to herein, i.e. within the interval of base positions 20156961 and 22499122 on bovine chromosome 21 (Btau_4.0). Further provided are methods wherein such a fingerprint may be generated by RFLPs of DNA or RNA obtained from the bovine subject, optionally the DNA or RNA may be amplified prior to analysis, the methods of which are well known in the art. RNA fingerprints may be performed by, for example, digesting an RNA sample obtained from the subject with a suitable RNase, or a ribozyme and, for example, electrophoretically separating and detecting the RNA fragments.

Furthermore, the possibility to detect animals that are carriers of BS can be utilized very effectively for marker assisted selection to enhance fertility. We have indeed demonstrated in the present invention that carrier-status for BS is strongly correlated with fertility, one of the most important economic traits in cattle. Indeed, only eight calves affected with Brachyspina have been described in the Holstein-Friesian dairy cattle population to date. However, the D allele is much more common than might be expected from the apparently low incidence of the disease. We demonstrate in this invention that +/D carrier sires are characterized by a highly significant decrease in "non-return rate". This is due to the fact that when mated to +/D cows (estimated to represent 10% of the HF population), one quarter of the embryos are affected D/D and will die early in development causing the cow to abort and "return in heat". Thus Brachyspina is a much more important issue in cattle than reflected by the incidence of calves born affected. Detecting /D carriers can thus be used for marker assisted selection to enhance fertility.

As a result of this invention, it is now possible to detect +/D carrier animals for BS by means of simple genetic tests performed on a nucleic acid extracted from biological samples originating from said animals and use the information obtained by the methods of the present invention for marker assisted selection for increased fertility.

The term "marker assisted selection for increased fertility" in accordance with the present invention refers to the use of "marker information" corresponding in fact to genotypes for SNPs located between nucleotide positions 20156961 and 22499122 on bovine chromosome 21 (Btau_4.0) to identify animals that are carriers of the BS risk haplotype following the procedures described above, and thereby obtain information about their breeding value for phenotypes related to male or female fertility. It is noteworthy that a novel form of marker assisted selection has been recently introduced referred to as "genomic selection" (see for instance reference 7). Information about the presence or absence of the BS risk haplotype will be utilized if the genomic selection procedure were to be applied for traits related to both male and female fertility. Genomic selection for such traits would thus utilize information that is disclosed for the first time in the present invention.

### Brief description of the figures

**Figure 1****:** Illustration of the genetic principles underlying the use of diagnostic haplotypes to detect BS carriers. **A**. Identification of the diagnostic haplotype as a region of homozygosity shared by all affected individuals. **B**. "Phasing" on the basis of parental information. Unphased genotypes are experimentally determined on a trio (father, mother and offspring) and shown as "x,y". Examination of the trio's genotype for a given SNP allows sorting of the offspring's allele according to parental origin which equates to "phasing". The outcome (phased genotypes) is boxed, allowing identification of the diagnostic haplotype in the offspring demonsytrating that he is BS carrier. **C**. Use of unphasqed genotypes to detect carriers. The "R(ejected)" genotypes, incompatible with carrier stataus are shown in the unphased individuals allowing exclusion of carrier status for individual "A" but not "B".
**Figure 2****:** Newborn Holstein-Friesian calf with Brachyspina.

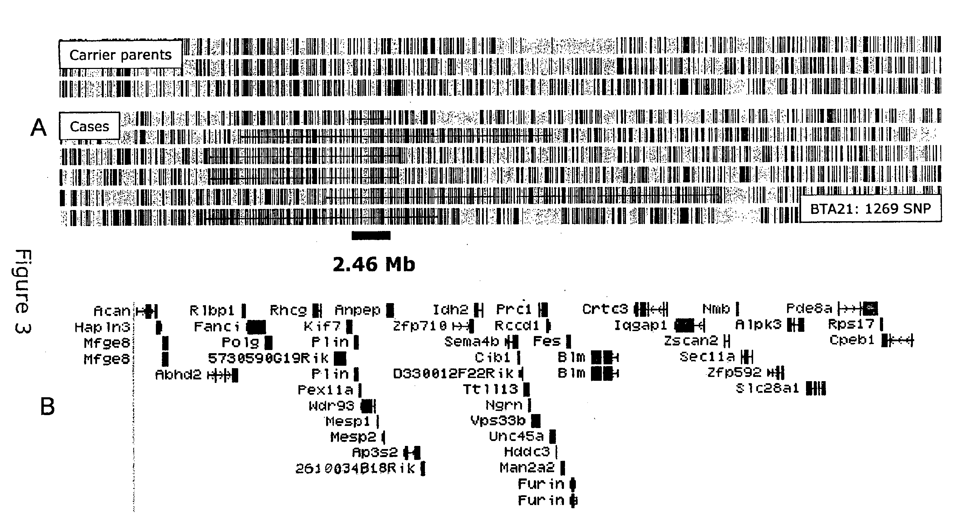

**Figure 3****:** Autozygosity mapping of the Brachyspina locus. **A**. Genotypes of cases and controls for chromosome 21 SNPs. Homozygous genotypes are shown in grey (yellow) or white, heterozygous genotypes in black (red). Overlapping blocks of extended homozygosity in the cases are shown in black and gray marked with a line. The limits and size of the haplotype shared homozygous and identical-by-state amongst all cases is highlighted in black (red). **B**. Gene content of the shared chromosome segment.

SEQ ID N°1: Nucleotide sequence for base positions 20156961 to 22499122 of bovine chromosome 21 (Btau_4.0).

### Detailed description of the invention

### Mapping the gene and mutation causing BS to BTA21.

To position the gene causing BS, we collected tissue samples from six affected individuals in the Holstein-Friesian dairy cattle population. Samples originated from the Netherlands, Denmark and Italy. DNA was extracted from the tissue samples and genotyped using a previously described panel of 60,000 bovine SNPs (HG 60K panel)(3), alongside control samples from healthy control individuals from the same breed. The ensuing genotypes were examined visually as well as with the previously described ASSHOM and ASSIST (3) programs. A chromosomal region spanning 2.46 Mb shared homozygous by descent by the six affected individuals was readily identified. The haplotype sharing was shown to be highly significant. The critical region, bounded by the nearest recombinational events, encompassed 56 annotated genes (Figure 3).

### Defining a diagnostic SNP haplotype for BS using the HG 60 K panel (3).

Table 1 shows the haplotype (obtained with the HG 60K SNP panel (3)) shared homozygous by all cases within the critical region. To examine the sensitivity and specificity of this haplotype in identifying BS +/D carriers, we genotyped 1999 Dutch Holstein-Friesian animals with the same SNP panel. The BTA21 genotypes were phased using purpose-build software and the animals carrying the BS-haplotype on one of their chromosomes identified. 193 such animals were observed corresponding to a BS carrier frequency of 9.7%. Pedigree analysis showed that all BS-carriers traced back to a common ancestor ("Sweet-Haven Tradition TM"), while 186 of them traced back to Sweet-Haven Tradition's son: "Bis-May Tradition Cleitus". These very popular sires must have spread the defect in the general population because of their extensive use by artificial insemination.

### Effect of BS carrier status on fertility.

The 9.7% incidence of BS-haplotype carriers, including some popular sires, suggested that BS should be much more common than observed. Most haplotype carriers were actually not known to be BS carriers. We reasoned that this might be due to the fact that the majority of BS cases would abort prematurely. To test this hypothesis we examined the non- return ("in heat") rate after insemination with semen from putative BS-carriers carrying the diagnostic haplotype. A highly significant effect of BS-haplotype on "non return rate" was indeed observed (Table2). The significance of the effect was highest for non return rate at 270 days post-insemination, suggesting that many affected foetuses die relatively late during gestation. The BS D allele is thus a major determinant not only of the BS defect but also of fertility, one of the economically most important traits in cattle.

### Specificity and sensitivity of the BS-diagnostic haplotype without phasing.

In a number of instances it may not be possible to reliably phase the SNP genotypes of certain individuals. To test whether the BS-specific haplotype could be used as a diagnostic in the absence of phasing we attempted to detect putative carriers in the same population using their unphased SNP genotypes. The sensitivity of the test (probability to detect true carriers) was 100%, while the specificity was 193/197 = 98%. Only 4 out of 1,806 non-carriers were wrongly labelled as carriers. The minimum number of SNPs needed to achieve this sensitivity and specificity was 7.

### Defining additional diagnostic SNPs on the BS critical region.

The 55 SNPs of the HG 60K panel mapping to the BS critical region defined as described above, are of course only a fraction of all SNP mapping in that interval. To identify additional diagnostic SNP, and thereby increase the sensitivity and specificity of the BS-diagnostic haplotype we genotyped six cases and 20 controls for the public domain 60K panel (6). Using the same approach as described before, we identified a shared region of homozygosity coinciding with the previously defined location. Table 1 lists all SNP in the BS-critical region with the corresponding allele on the BS-specific haplotype. The SNPs listed in Table 1 can all be used in isolation or preferably in conjunction to define the BS-carrier status of animals.

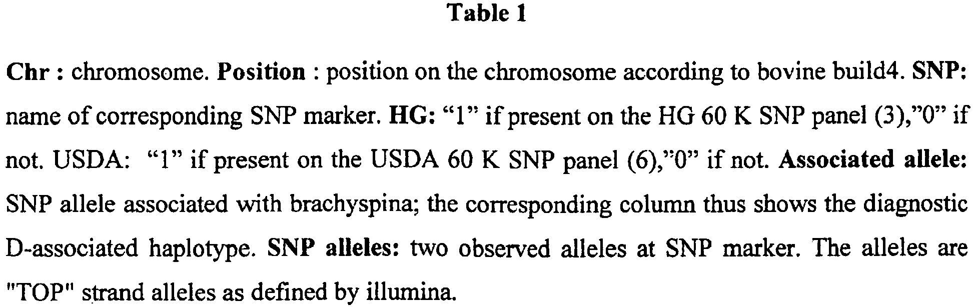

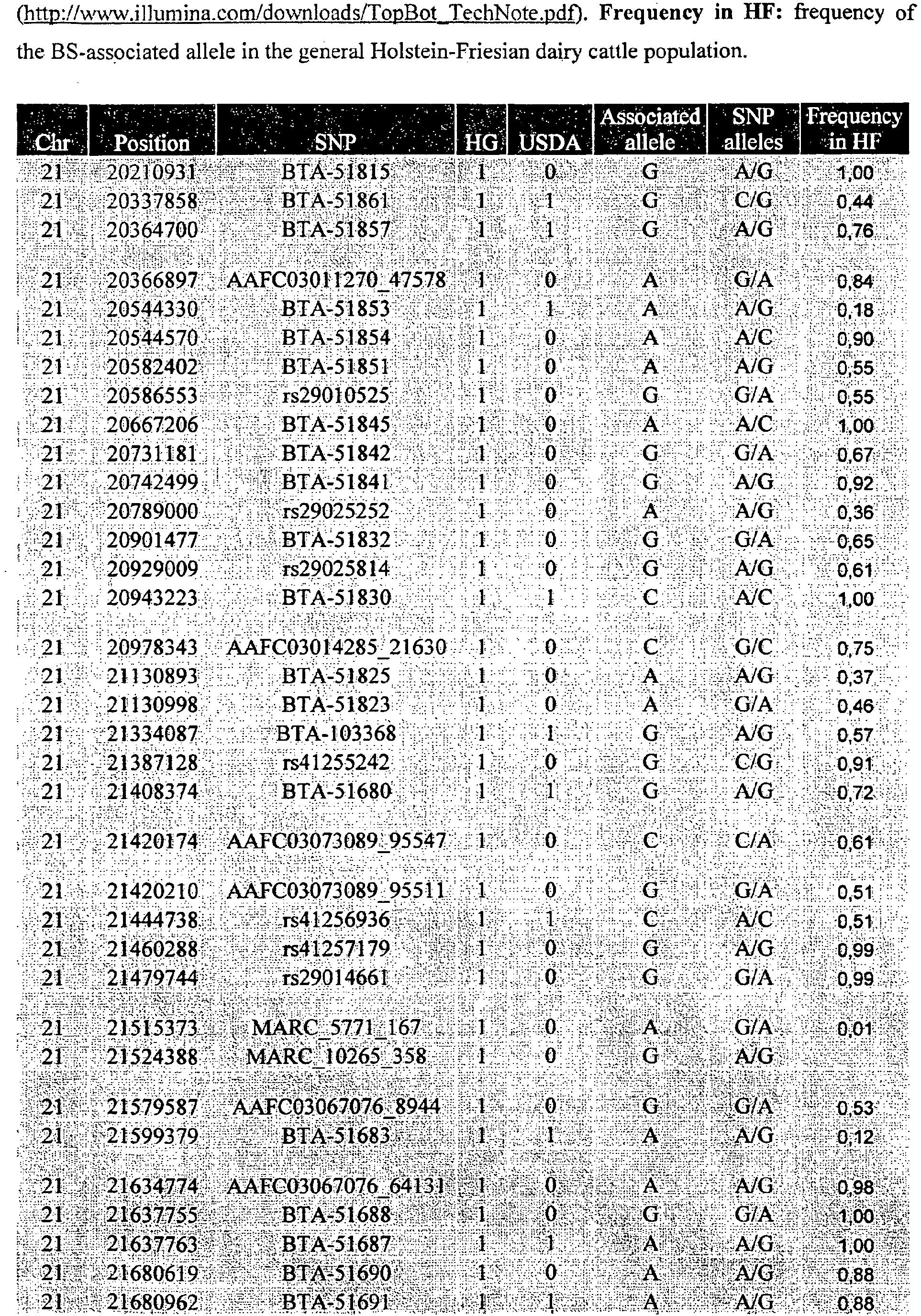

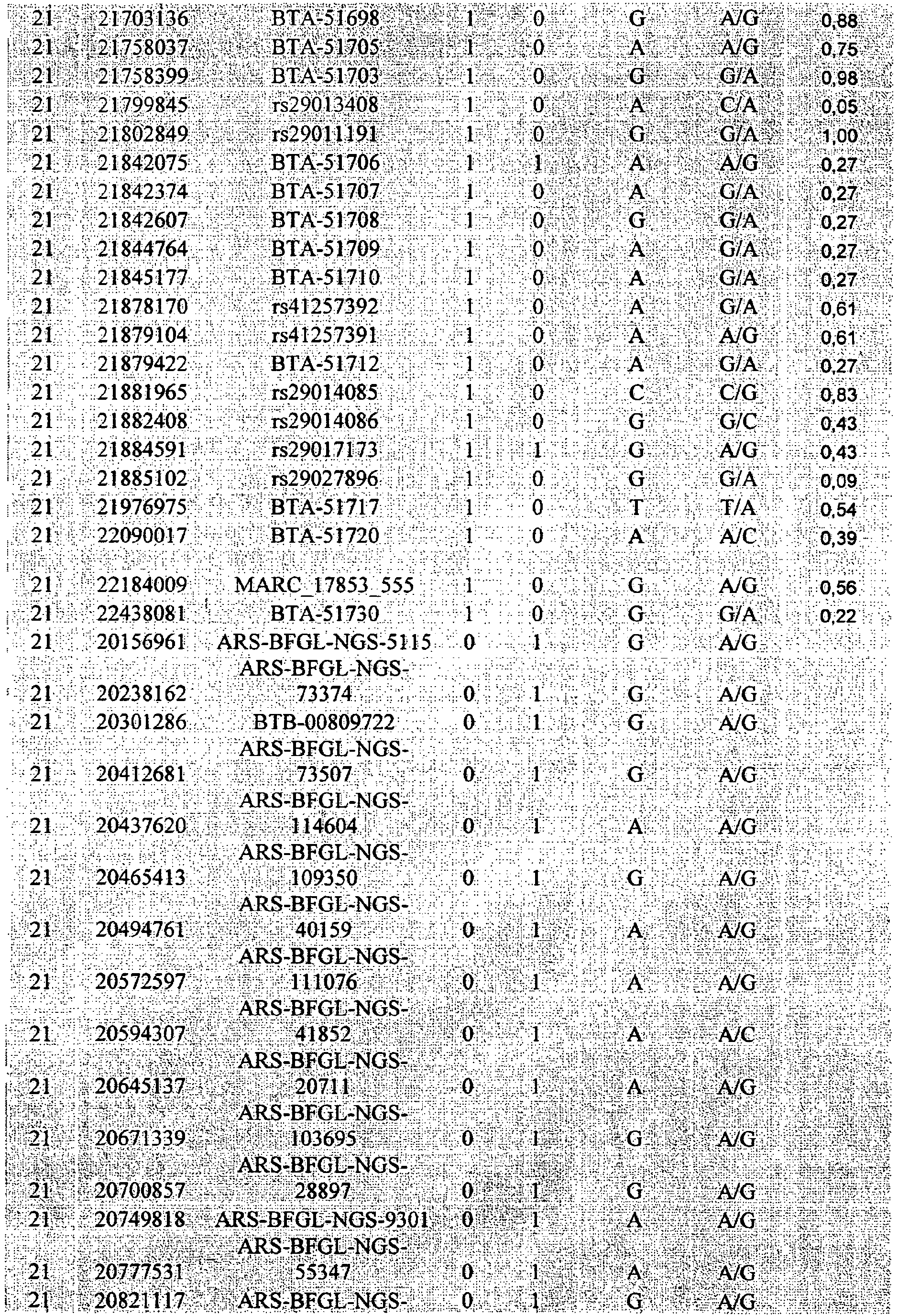

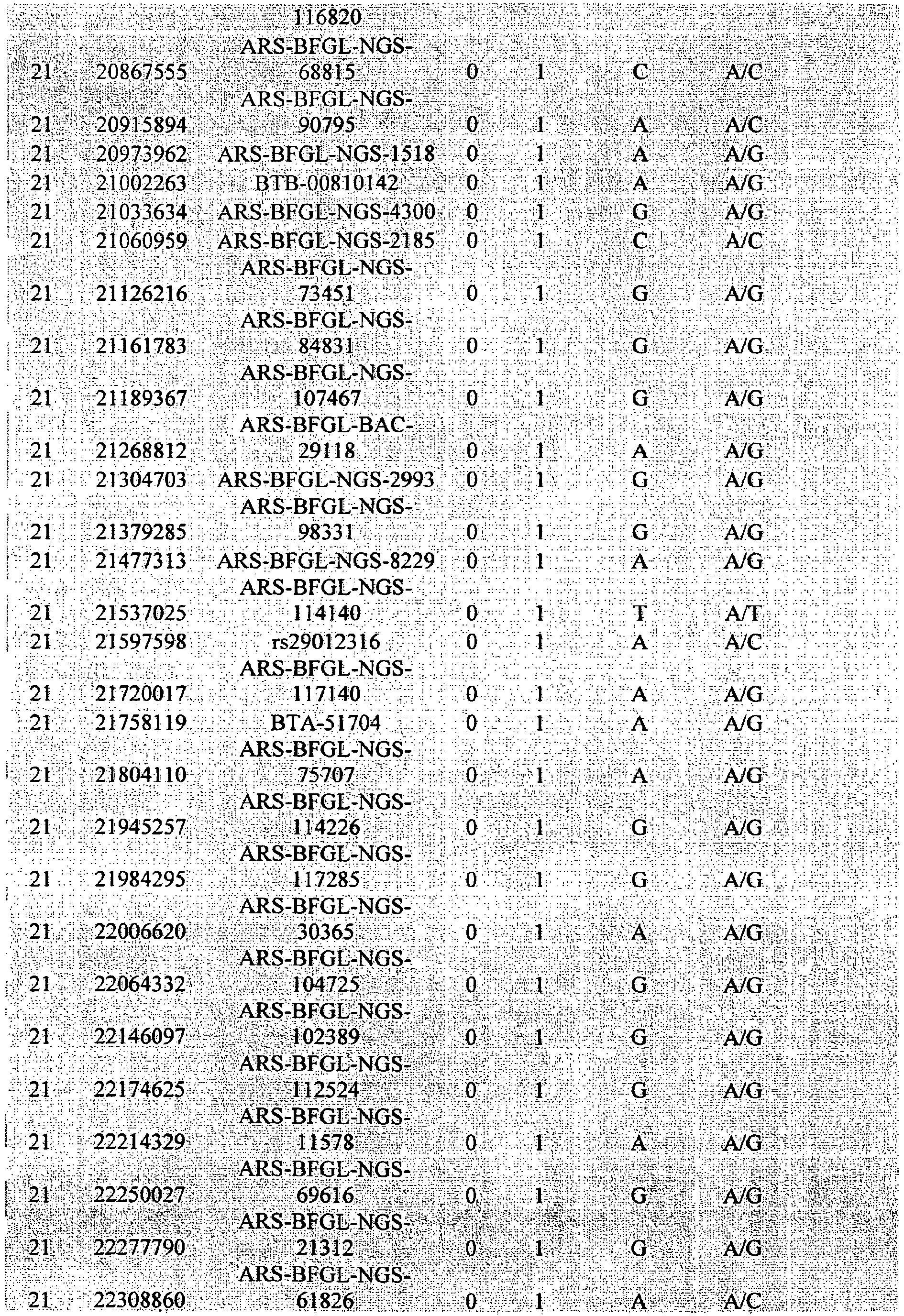

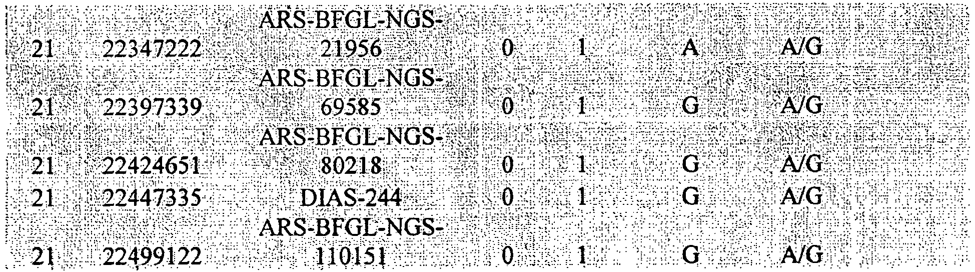

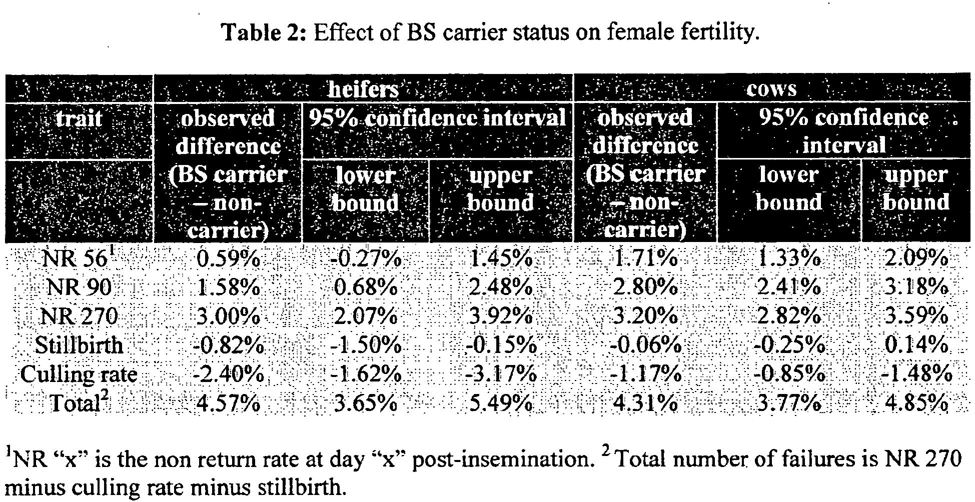

### References

1. Shuster DE, Kehrli ME Jr, Ackermann MR, Gilbert RO. (1992) Identification and prevalence of a genetic defect that causes leukocyte adhesion deficiency in Holstein cattle. Proc Natl Acad Sci U S A. 89:9225-9229.
2. Thomsen B, Horn P, Panitz F, Bendixen E, Petersen AH, Holm LE, Nielsen VH, Agerholm JS, Arnbjerg J, Bendixen C. (2006) A missense mutation in the bovine SLC35A3 gene, encoding a UDP-N-acetylglucosamine transporter, causes complex vertebral malformation. Genome Res. 16:97-105. Epub 2005 Dec 12.
3. Charlier C, Coppieters W, Rollin F, Desmecht D, Agerholm JS, Cambisano N, Carta E, Dardano S, Dive M, Fasquelle C, Frennet JC, Hanset R, Hubin X, Jorgensen C, Karim L, Kent M, Harvey K, Pearce BR, Simon P, Tama N, Nie H, Vandeputte S, Lien S, Longeri M, Fredholm M, Harvey RJ, Georges M. (2008) Highly effective SNP-based association mapping and management of recessive defects in livestock. Nat Genet. 40:449-54. Epub 2008 Mar 16.
4. Agerholm J.S. and Peperkamp K. (2007) Familial occurrence of Danish and Dutch cases of the bovine brachyspina syndrome. BMC Vet Res. 3:8.
5. Syvanen, A.C. (2001) Accessing genetic variation: genotyping single nucleotide polymorphisms. Nature Reviews Genetics 2: 930-942.
6. Van Tassell CP, Smith TP, Matukumalli LK, Taylor JF, Schnabel RD, Lawley CT, Haudenschild CD, Moore SS, Warren WC, Sonstegard TS. (2008) SNP discovery and allele frequency estimation by deep sequencing of reduced representation libraries. Nat Methods 5:247-52. Epub 2008 Feb 24.
7. Meuwissen TH, Hayes BJ, Goddard ME. (2001) Prediction of total genetic value using genome-wide dense marker maps. Genetics. 2001 Apr;157(4):1819-29.

## Claims

1. A method for determining whether a bovine is affected by or a carrier of brachyspina (BS) by analyzing its genomic DNA, the method comprising the steps of:
a) extracting the DNA from a sample of biological material containing said genomic DNA obtained from the bovine,
b)i) genotyping said DNA for SNPs located in the interval between nucleotide positions 20156961 and 22499122 on bovine chromosome 21, Btau_4.0, and
c) ascertaining whether said animal carries the brachyspina-risk haplotype.

2. A method for determining whether a bovine is affected by or a carrier of brachyspina (BS) by analyzing its RNA, the method comprising the steps of :
a) extracting the RNA from a sample of biological material containing said RNA obtained from the bovine,
b)i) genotyping said RNA for SNPs located in the interval between nucleotide positions 20156961 and 22499122 on bovine chromosome 21, Btau_4.0, and
c) ascertaining whether said animal carries the brachyspina-risk haplotype.

3. The method of claim 1 or 2 further comprising the step of:
b)ii) phasing the SNP genotype of said bovine.

4. The method of any one of claims 1 to 3, wherein the SNPs utilized are as defined in Table 1.

5. The method of any of claims 1 to 4, wherein the identification of at least one SNP is indicative for brachyspina in said bovine.

6. The method of any one of claims 1 to 4, wherein the identification of at least 5 SNPs as defined in Table 1 are indicative for brachyspina in said bovine.

7. The method of any one of claims 1 to 6, wherein the sample is selected from the group consisting of blood, sperm, hair roots, milk, body fluids and/or tissues including nucleated cells.

8. The method of any one of claims 1 to 7, wherein the bovine is selected from the species bos taurus.

9. The method of claim 8 wherein the bovine is selected from the group consisting of Holstein, Friesian and Holstein-Friesian Cross breeds, British and /or Dutch Friesian.

10. Use of the method of any one of claims 1 to 9 to perform marker assisted selection or genomic selection for increased fertility in said bovine.

## Patentansprüche

1. Verfahren zum Bestimmen, ob ein Rind von Brachyspina (BS) befallen ist oder ein Träger von Brachyspina (BS) ist, durch Analyse seiner genomischen DNA, wobei das Verfahren die folgenden Schritte umfasst:
a) Extrahieren der DNA aus einer Probe von die genomische DNA enthaltendem biologischen Material, die von dem Rind erhalten wurde,
b)i) Genotypisieren der DNA auf SNPs, die in dem Abschnitt zwischen Nucleotidpositionen 20156961 und 22499122 auf dem Rinderchromosom 21, Btau_4.0 lokalisiert sind, und
c) Feststellen, ob das Tier den Brachyspinarisiko-behafteten Haplotyp trägt.

2. Verfahren zum Bestimmen, ob ein Rind von Brachyspina (BS) befallen ist oder ein Träger von Brachyspina (BS) ist, durch Analyse seiner genomischen RNA, wobei das Verfahren die folgenden Schritte umfasst:
a) Extrahieren der RNA aus einer Probe von die genomische RNA enthaltendem biologischen Material, die von dem Rind erhalten wurde,
b)i) Genotypisieren der RNA auf SNPs, die in dem Abschnitt zwischen Nucleotidpositionen 20156961 und 22499122 auf dem Rinderchromosom 21, Btau_4.0 lokalisiert sind, und
c) Feststellen, ob das Tier den Brachyspinarisiko-behafteten Haplotyp trägt.

3. Verfahren nach Anspruch 1 oder 2, das des Weiteren den folgenden Schritt umfasst:
b)ii) Phasing des SNP-Genotyps des Rindes.

4. Verfahren nach einem der Ansprüche 1 bis 3, wobei die verwendeten SNPs wie in Tabelle 1 definiert sind.

5. Verfahren nach einem der Ansprüche 1 bis 4, wobei die Identifikation von mindestens einem SNP ein Indikator für Brachyspina in dem Rind ist.

6. Verfahren nach einem der Ansprüche 1 bis 4, wobei die Identifikation von mindestens 5 wie in Tabelle 1 definierten SNPs ein Indikator für Brachyspina in dem Rind ist.

7. Verfahren nach einem der Ansprüche 1 bis 6, wobei die Probe ausgewählt ist aus der Gruppe bestehend aus Blut, Sperma, Haarwurzeln, Milch, Körperflüssigkeiten und/oder Geweben einschließlich kernhaltiger Zellen.

8. Verfahren nach einem der Ansprüche 1 bis 7, wobei das Rind aus der Art Bos taurus ausgewählt ist.

9. Verfahren nach Anspruch 8, wobei das Rind ausgewählt ist aus der Gruppe bestehend aus Holstein, Friesian und Holstein-Friesian-Kreuzungen, Britisch- und/oder Holländisch-Friesian.

10. Verwendung des Verfahrens nach einem der Ansprüche 1 bis 9, um eine Markerunterstützte Selektion oder genomische Selektion für erhöhte Fruchtbarkeit in dem Rind durchzuführen.

## Revendications

1. Procédé permettant de déterminer si un bovin est affecté ou non par le brachyspina (BS) ou en est porteur en analysant son ADN génomique, le procédé comprenant les étapes consistant à :
a) extraire l'ADN à partir d'un échantillon de matériel biologique contenant ledit ADN génomique obtenu auprès du bovin,
b)i) établir le génotype dudit ADN pour identifier les SNP situés dans l'intervalle entre les positions des mononucléotides 20156961 et 22499122 sur le chromosome bovin 21, Btau_4.0, et
c) vérifier si ledit animal est porteur de l'haplotype du risque de brachyspina ou ne l'est pas.

2. Procédé permettant de déterminer si un bovin est affecté ou non par le brachyspina (BS) ou en est porteur en analysant son ARN, le procédé comprenant les étapes consistant à :
a) extraire l'ARN à partir d'un échantillon de matériel biologique contenant ledit ARN obtenu auprès du bovin,
b)i) établir le génotype dudit ADN pour identifier les SNP situés dans l'intervalle entre les positions des mononucléotides 20156961 et 22499122 sur le chromosome bovin 21, Btau_4.0, et
c) vérifier si ledit animal est porteur de l'haplotype du risque de brachyspina ou ne l'est pas.

3. Procédé selon la revendication 1 ou 2, comprenant en outre l'étape consistant à :
b)ii) reconstruire le génotype des SNP dudit bovin.

4. Procédé selon l'une quelconque des revendications 1 à 3, dans lequel les SNP utilisés sont tels qu'ils sont définis dans le tableau 1.

5. Procédé selon l'une quelconque des revendications 1 à 4, dans lequel l'identification d'au moins un SNP indique la présence du brachyspina chez ledit bovin.

6. Procédé selon l'une quelconque des revendications 1 à 4, dans lequel l'identification d'au moins 5 SNP tels qu'ils sont définis dans le tableau 1 indique la présence du brachyspina chez ledit bovin.

7. Procédé selon l'une quelconque des revendications 1 à 6, dans lequel l'échantillon est sélectionné dans le groupe constitué par le sang, le sperme, les racines des poils, le lait, les fluides corporels et/ou d'autres tissus comprenant des cellules nucléées.

8. Procédé selon l'une quelconque des revendications 1 à 7, dans lequel le bovin est sélectionné dans l'espèce Bos taurus.

9. Procédé selon la revendication 8, dans lequel le bovin est sélectionné dans le groupe constitué par la race holstein, la race frisonne et les croisements entre la race frisonne et la race holstein, la race britannique et/ou la race néerlandaise.

10. Utilisation du procédé selon l'une quelconque des revendications 1 à 9 pour réaliser une sélection assistée par marqueurs ou une sélection génomique en vue d'une fertilité accrue chez ledit bovin.
